# EUROPEAN PATENT APPLICATION

(11) **EP 1 685 897 A2**
(43) Date of publication of application: **02.08.2006**
(21) Application number: 04793400.5
(22) Date of filing: 29.10.2004
(51) Int. Cl.: B01J 29/40, C01B 39/38, C07B 61/00, C07C 1/24, C07C 11/02, C10G 3/00

(54) **CATALYST, PROCESS FOR PREPARING THE CATALYST AND PROCESS FOR PRODUCING LOWER HYDROCARBON WITH THE CATALYST**

(30) Priority: 05.11.2003 JP 2003375666
(71) Applicant: JGC Corporation, Tokyo 100-0004 (JP)
(72) Inventor: ITO, Hirofumi c/o JGC Corp. Research & Development, Ibaraki-gun, Ibaraki 313-1313 (JP); OOYAMA, Koji JGC Corp. Research & Development Cntr, Ibaraki-gun, Ibaraki 313-1313 (JP); YAMADA, Syoichi JGC Corp. Research & Devel. Cntr, Ibaraki-gun, Ibaraki 313-1313 (JP); KUME, Motohisa JGC Corp. Research & Devel. Center, Ibaraki-gun, Ibaraki 313-1313 (JP); CHIKAMATSU, Nobuyasu JGC Corp. Research & Dev.Cntr, Ibaraki-gun, Ibaraki 313-1313 (JP)
(74) Representative: Poulin, Gérard
(86) International application number: PCT/JP2004/016480
(87) International publication number: WO 2005/044760

(57) **Abstract**

An alkaline earth metal-containing MFI zeolite catalyst is used when synthesizing a lower hydrocarbon from dimethyl ether and/or methanol, which has a Si/Al atomic ratio ranging from 30 to 400, an alkaline earth metal/Al atomic ratio ranging from 0.75 to 15, and an average particle diameter ranging from 0.05 to 2 µm.

## Description

### TECHNICAL FIELD

The present invention relates to a zeolite catalyst used in a process for synthesizing a lower hydrocarbon by a dehydration reaction from dimethyl ether and/or methanol, a preparation process of the catalyst, and a process for producing a lower hydrocarbon using the catalyst.

Priority is claimed on Japanese Patent Application No. 2003-375666, filed November 5, 2003, the content of which is incorporated herein by reference.

### BACKGROUND ART

Since a method for synthesizing a hydrocarbon which contains gasoline as a principal component from methanol or dimethyl ether using a zeolite catalyst was proposed by U.S. Mobil Oil Corp., research into the preparation of a synthetic zeolite catalyst and a process for producing a hydrocarbon using the same catalyst has advanced. As the preparation process of the synthetic zeolite catalyst, a so-called "hydrothermal synthesis method", which comprises mixing the component as the raw material of zeolite to form a raw material solution, and thereafter heating the resultant raw material solution at a high temperature to form crystals in the mixed raw material solution, is generally known.

A process for producing an olefin using an alkaline earth metal-containing zeolite catalyst and the above catalyst has been proposed in Patent document 1(Japanese Post.-Exam.Publication No. S. 63-35570 Official Report). Among these synthetic zeolite catalysts which are prepared by this hydrothermal synthesis method, that process uses the catalyst for synthesizing a lower unsaturated hydrocarbon such as ethylene, propylene, etc., from dimethyl ether and/or methanol.

However, the catalyst disclosed in Patent document 1 has a problem in that the catalyst life is short, because carbonaceous materials are deposited on the surface of the catalyst so that the active sites (acid sites, etc.), which effectively act on synthesis reaction of a lower hydrocarbon, are poisoned.

### DISCLOSURE OF INVENTION

In view of the problem of the above conventional technology, the present invention was made. That is, it is an object of the present invention to provide a zeolite catalyst with a small average particle size, being capable of synthesizing a lower hydrocarbon at a high yield from dimethyl ether and/or methanol, having an extended catalyst life, and being an alkaline earth metal-containing MFI zeolite catalyst, and to provide a preparation process therefor and a process for producing a lower hydrocarbon using the catalyst.

A first aspect of the present invention is an alkaline earth metal-containing MFI zeolite catalyst for use in synthesizing a lower hydrocarbon from dimethyl ether and/or methanol, having a Si/Al atomic ratio ranging from 30 to 400, an alkaline earth metal/Al atomic ratio ranging from 0.75 to 15, and an average particle diameter ranging from 0.05 to 2 µm.

A second aspect of the present invention is a process for preparing an alkaline earth metal-containing MFI zeolite catalyst for use in synthesizing a lower hydrocarbon from dimethyl ether and/or methanol, comprising synthesizing a zeolite raw material solution which contains an SiO₂ source, a metal oxide source, an alkaline source, and a structure directing agent (SDA), in the presence of an alkaline earth metal salt and a zeolite seed crystal.

A third aspect of the present invention is a process for producing a lower hydrocarbon comprising synthesizing a lower hydrocarbon from dimethyl ether and/or methanol, in which the alkaline earth-metal containing MFI zeolite catalyst as set forth in the above is employed, the lower hydrocarbon is an unsaturated hydrocarbon having 2 to 4 carbon atoms, and the yield of the carbon atoms contained in the lower hydrocarbon to the carbon atoms contained in said supplied dimethylether and/or said methanol is not less than 60% by weight.

In accordance with the zeolite catalyst of the present invention and the process for preparing the catalyst, an alkaline earth metal-containing MFI zeolite catalyst, which has a small average particle diameter of the catalyst and an extended catalyst life, can be provided. And by employing this catalyst in the case of synthesizing a lower hydrocarbon from dimethyl ether and/or methanol, an unsaturated hydrocarbon having 2 to 4 carbon atoms can be obtained at a high yield, and the catalyst life is extended so that the regeneration period of the catalyst can be elongated to reduce the number of regeneration, and as a result, production efficiency is increased and production cost can be decreased.

### BRIEF DESCRIPTION OF THE DRAWINGS

FIG. 1 is a scanning electron microscopic photograph of the synthetic zeolite of Example 1.
FIG. 2 is a scanning electron microscopic photograph of the synthetic zeolite of Comparative Example 1.

### BEST MODE FOR CARRYING OUT THE INVENTION

Hereafter, suitable examples of the present invention will be explained, referring to drawings. However, the present invention is not limited to each of the following examples, and for example, it is possible to combine suitably the constitutional elements of these examples with each other.

In an alkaline earth metal-containing MFI zeolite catalyst of the present invention, an Si/Al atomic ratio ranges from 30 to 400, an alkaline earth metal/Al atomic ratio ranges from 0.75 to 15, and an average particle diameter ranges from 0.05 to 2 µm.

Note, the MFI is a framework type code which is defined by the international zeolite association (IZA).

In the alkaline earth metal-containing MFI zeolite catalyst of the present invention, the atomic ratio of Si and Al (Si/Al) ranges from 30 to 400. If the Si/Al atomic ratio is less than 30, then deposition of carbonaceous material on the catalyst is promoted by increasing effective acid sites, thereby causing early deactivation of the catalyst, whereas if the Si/Al atomic ratio exceeds 400, then decrease of the catalytic activity is caused by reduction of effective acid sites.

Moreover, in the alkaline earth metal-containing MFI zeolite catalyst of the present invention, the atomic ratio (M/Al) of an alkaline earth metal (it will be referred to as "M" hereinafter) and Al ranges from 0.75 to 15. If the M/Al atomic ratio is less than 0.75, then the catalyst life will decrease, and the yield of the lower unsaturated hydrocarbon synthesized from dimethyl ether and/or methanol will decrease. On the other hand, if the M/Al atomic ratio exceeds 15, then it becomes difficult to prepare a zeolite catalyst. By making an MFI zeolite catalyst contain an alkaline earth metal, a lower unsaturated hydrocarbon having a small carbon number can be synthesized from dimethyl ether and/or methanol.

Furthermore, in the alkaline earth metal-containing MFI zeolite catalyst of the present invention, an average particle diameter ranges from 0.05 to 2 µm. Among them, the average particle diameter ranges preferably from 0.1 to 1.5µm, more preferably from 0.1 to 1µm. Catalyst life can be extended by decreasing the catalyst average particle diameter.

The alkaline earth metal-containing MFI zeolite catalyst of the present invention is a proton type. By making a catalyst be a proton type, the catalytic activity of lower hydrocarbon production increases. The process for preparing an alkaline earth metal-containing MFI zeolite catalyst of the present invention consists of a mixing step of mixing a zeolite raw material solution, while making an alkaline earth metal salt and a zeolite seed crystal coexistent therein; a synthesizing step of heating the resultant mixed solution in an autoclave under a self-pressure to perform hydrothermal treatment; and a step of drying and calcining the resultant product after the hydrothermal synthesis.

### [Preparation of zeolite raw material solution]

A SiO₂ source, a metal oxide source, an alkali source, and SDA are contained in the zeolite raw material solution used in the present invention. As a SiO₂ source in the zeolite raw material solution component, water glass, silica sol, silica gel, silica, etc. are exemplary. Among them, water glass and silica sol are preferable. Moreover, each of these SiO₂ sources may be used solely or in combination.

As a metal oxide source in the zeolite raw material solution component used in the present invention, a water-soluble metal salt, which consists of an aluminium, a titanium, a gallium, etc. is exemplary. Among them, a trivalent metal oxide is preferable, and an aluminium salt is more preferable. Specifically, aluminium nitrate, aluminium sulfate, sodium aluminate, and alumina sol etc. are exemplary. Moreover, these metal oxide sources may be used solely or in combination of two or more thereof.

As an alkali source in the zeolite raw material solution component used in the present invention, sodium oxide in water glass, sodium hydroxide, potassium hydroxide, sodium aluminate, sodium chloride, potassium chloride, etc. are exemplary. Each of these alkali sources may be used solely or in combination of two or more thereof.

The SDA in the zeolite raw material solution component used in the present invention is a component which is added in order to synthesize the zeolite catalyst having a desired framework type. Specifically, as such SDA for synthesizing an MFI zeolite catalyst, tetrapropyl ammonium compounds are exemplary. Among them, tetrapropyl ammonium bromide is preferable.

Each of the sources for the zeolite raw material solution and water are mixed in a desirable proportion, so that a zeolite raw material solution is prepared.

Note, an acid source other than the above component, such as sulfuric acid and nitric acid, etc. can be added to the zeolite raw material solution used in the present invention.

As an alkaline earth metal salt which is to be contained in the zeolite raw material solution used in the present invention, organic salts such as acetates and propionate of calcium, strontium, magnesium, and barium etc., and inorganic salts such as chlorides and nitrates of these metals are exemplary. Among them, in view of the yield of lower hydrocarbons and the catalyst life, a calcium salt and a strontium salt are preferable, and calcium acetate is more preferable. As to the content of an alkaline earth metal salt, an alkaline earth metal salt is added so that the alkaline earth metal/Al atomic ratio in the synthetic zeolite catalyst should range from 0.75 to 15, as stated above. In the present invention, the zeolite catalyst modified with an alkaline earth metal can be obtained by mixing an alkaline earth metal salt with the zeolite raw material solution, and then performing hydrothermal synthesis thereon.

As a zeolite seed crystal which is coexistent with the zeolite raw material solution of the present invention, zeolite having a framework such as a Ferrierite structure, Moldenite structure, MFI structure, etc. are exemplary. Among them, in view of ease and stability of crystallization, zeolite having an MFI structure is preferable.

Moreover, the amount of zeolite seed crystal added corresponds to 1 to 60 mass % of the amount of alkaline earth metal-containing MFI zeolite catalyst which is synthesized without adding a zeolite seed crystal. If the amount of the zeolite seed crystal added is less than 1 mass%, then it is impossible to make a synthetic zeolite catalyst fine, whereas if it exceeds 60 mass%, then the content of the alkaline earth metal-containing zeolite in the synthetic zeolite catalyst will decrease, and hence it is not preferable in terms of performance.

Moreover, as to the size of the zeolite seed crystal, an average particle diameter thereof is preferably not more than 1.5 µm, more preferably not more than 0.5 µm. An average particle diameter of the synthetic zeolite catalyst can be made to be within a range of 0.05 to 2 µm, and a catalyst life can be extended by making a zeolite seed crystal having an average particle diameter of not more than 1.5 µm be coexistent in the zeolite raw material solution.

Moreover, in order to disperse the seed crystal efficiently in the raw material solution when using a seed crystal, it is effective to perform treatment, such as application of ultrasonic wave, jet-milling etc., on the seed crystal in advance, so that the particle which is made of aggregated crystal can be dispersed independently as much as possible.

### [Preparation step of a zeolite catalyst]

The preparation step of an alkaline earth metal-containing MFI zeolite catalyst of the present invention is as follows.

First, a zeolite raw material solution which contains 100 mol parts of SiO₂ source and 0.2 to 4.0 mol parts of a metal oxide source, 2 to 1000 mol parts of alkali source, and 2 to 200 mol parts of an SDA are dissolved in water, and a 0.1 to 60 mol parts of alkaline earth metal salt and a zeolite seed crystal in an amount which is equivalent to 1 to 60 mass% of quantity of the zeolite which is generated in the case of synthesizing without adding a zeolite seed crystal are added thereto, and the resultant mixture is stirred. Through this mixing step, the resultant mixed solution which contains the zeolite raw material solution, the alkaline earth metal salt, and the zeolite seed crystal becomes an aqueous gel mixture.

Subsequently, this aqueous gel mixture is transferred to an autoclave, and thereafter the aqueous gel mixture is heated and stirred under a self-pressure, at 60 to 250°C, for 1 to 200 hours, thereby performing hydrothermal synthesis. The product of the hydrothermal synthesis is separated by filtration or centrifugal separation and washed with water, and then dried and thereafter calcined at 300 to 700°C for 1 to 100 hours. Through these steps, an alkaline earth metal-containing MFI zeolite catalyst is prepared.

Note, in the case of making an alkaline earth metal-containing MFI zeolite catalyst be a proton type, a step of performing an acid treatment on the product or a step of performing ion exchange of the product into an ammonium type, and a step of drying and calcining again are added. In the acid treatment, inorganic acids such as hydrochloric acid, sulfuric acid, and nitric acid, and organic acids, such as formic acid, acetic acid, are used. Among them, hydrochloric acid is preferable. Moreover, the ion exchange for converting it into ammonium type is performed in an aqueous solution of ammonium salt, such as ammonia water, ammonium chloride, ammonium nitrate, and ammonium sulfate, etc.

### [Production method for a lower hydrocarbon]

In order to synthesize a lower hydrocarbon from dimethyl ether and/or methanol using the alkaline earth metal-containing MFI zeolite catalyst obtained by the present invention, dimethyl ether and/or methanol are supplied as gas, thereby making them come into contact with the above catalyst. Specifically, a fixed bed reaction system, fluidized bed reaction system, etc. are exemplary.

As to a reaction, it can be performed under a wide range of conditions of temperature and pressure. Among them, the reaction temperature ranges preferably from 300 to 750°C, more preferably from 400 to 650°C. If the temperature is less than 300°C, then activity of the catalyst is insufficient, although it is advantageous in energy. On the other hand, if the temperature exceeds 750°C, then the coking rate is so high that deactivation is rapid and the irreversible deactivation of catalyst (collapse of zeolite framework,etc.) will occur. Dimethyl ether and/or methanol used as a raw material can be supplied onto the catalyst, with steam, inert gas, etc. In the case of performing the reaction continuously on a fixed bed reactor, the weight hourly space velocity (it will be referred as "WHSV"), which is a mass corresponding to dimethyl ether (it will be referred as "DME") to be supplied per unit catalyst mass and per unit time, preferably ranges from 0.025 to 50g-DME/(g-catalyst • hour). If WHSV is less than 0.025g-DME/(g-catalyst • hour), then the productivity of the reactor per unit volume is low, and hence it is not economical, whereas if WHSV exceeds 50g-DME/(g-catalyst • hour), then sufficient catalyst life and catalyst activity cannot be obtained. Moreover, the reactor effluent can be separated through a well-known separation refining process.

In the present invention, a lower hydrocarbon can be synthesized at a high yield from the raw material of dimethyl ether and/or methanol using the above alkaline earth metal-containing MFI zeolite catalyst. In accordance with the process for producing a lower hydrocarbon of the present invention, the yield of the unsaturated hydrocarbon having 2 to 4 carbon atoms, expressed in terms of mass basis of carbon contained in the supplied dimethyl ether and/or methanol becomes not less than 60%.

### Examples

Hereafter, the present invention will be explained in more detail, by showing Examples. Note, the present invention is not limited to the following examples at all.

### [Example 1]

### <Preparation of a zeolite catalyst>

A catalyst was prepared by an improved preparation method which was based on the preparation example of Patent Document 1 (Japanese Patent Publication No.S.63-35570 official report).

A zeolite raw material solution which consists of 9.50 g of A1 (NO₃)₃ • 9H₂O, and 10.92 g of Ca(CH₃COO)₂ • H₂O was dissolved in a 750 g of water. To the resultant solution, a solution in which 500 g of CATALOID SI-30 (produced by CATALYSTS & CHEMICALS IND. Co., Ltd.) water glass was dissolved in 333 g of water, 177.5 g of 6 mass % aqueous NaOH solution, 317.6 g of 21.3 mass% aqueous tetrapropyl ammonium bromide solution, and 15.0 g (which is equivalent to 10 mass% of the quantity of the zeolite catalyst which was synthesized without adding a seed crystal) of an ammonium type MFI zeolite (produced by Zeolyst International, having Si/Al atomic ratio of 70) having an average particle diameter of 0.5 µm as a zeolite seed crystal were added while stirring to obtain an aqueous gel mixture.

Subsequently, this aqueous gel mixture was put into a 3-L autoclave, and agitated at 160°C under a self-pressure for 18 hours to perform hydrothermal synthesis.

After filtering and washing the white solid product by hydrothermal synthesis, it was dried at 120°C for 5 hours, and was calcined at 520°C in air for 10 hours.

Thus calcined product was impregnated with 0.6 N hydrochloric acid, and the resultant mixture was stirred at room temperature for 24 hours, to exchange the Na ion of zeolite to proton.

Thereafter, the resultant product was dried at 120°C for 5 hours, after being filtered and washed with water, and was calcined at 520°C in air for 10 hours, to obtain a proton type alkaline earth metal-containing MFI zeolite catalyst.

The atomic ratio of Si and Al of Si/Al and the atomic ratio of Ca and Al of Ca/Al at the time of charging the raw material, and the atomic ratio of Si and Al of Si/Al and the atomic ratio of Ca and Al of Ca/Al in the synthetic zeolite catalyst are shown in Table 1.

Each atomic ratio at the time of charging the raw material was calculated from the purity and molecular weight of material. Moreover, each atomic ratio after synthesis was measured by X-ray fluorescence analysis device.

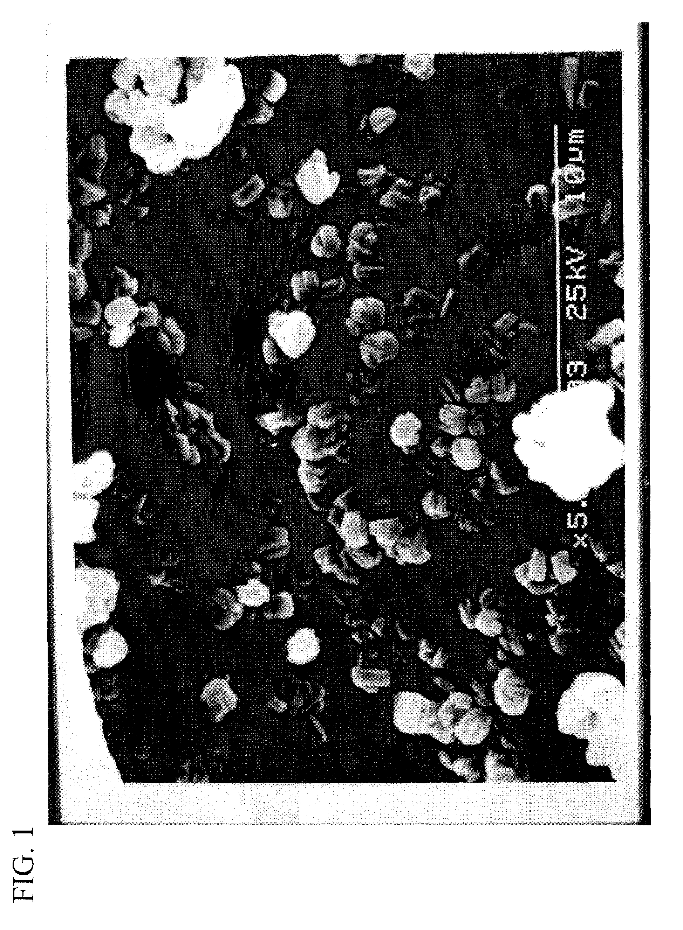

The average particle diameter of the prepared zeolite catalyst was measured by scanning electron microscope, and the specific surface area was measured by the BET adsorption method. "Average particle diameter (µm)" and "a specific surface area (m²/g)" of the catalyst are shown in Table 1. Moreover, scanning electron microscope photograph of the zeolite catalyst prepared in Example 1 is shown in FIG. 1.

### <Catalyst performance test>

In order to measure catalyst performance, a lower hydrocarbon was synthesized from dimethyl ether using the prepared proton type alkaline earth metal-containing MFI zeolite catalyst. The lower hydrocarbon was synthesized using a fixed bed type reactor. Dimethyl ether flowing at a rate of 637 Ncm³/hour and nitrogen flowing at a rate of 637 Ncm³/hour were mixed and fed to the reactor pipe, thereby reacting them with a catalyst at 530°C under atmospheric pressure. The weight hourly space velocity (WHSV), which is the ratio of the supplied quantity of dimethyl ether (DME) as a raw material to the quantity of the catalyst, was set to be 2.4 g-DME/(g-catalyst • hour).

The total mass of dimethyl ether which was fed to the catalyst per 1 g in the period from the time the reaction started to the time the conversion of DME and/or methanol became less than 99.9% was defined as "catalyst life." This unit is expressed in terms of "g-DME/g-catalyst". Moreover, "C2-C4 olefin yield(%)" was defined as the yield of an unsaturated hydrocarbon having 2 to 4 carbon atoms which is based on carbon atoms contained in the supplied dimethyl ether and/or methanol. Each component was measured by gas chromatography at 10 - 15 hours.

"Catalyst life (g-DME/g-catalyst)" and "C2-C4 olefin yield (%)" are shown in Table 1.

**[Table 1]**

| | | | Example 1 | Example 2 | Comparative Example 1 | Comparative Example 2 | Comparative Example 3 | Comparative Example 4 |
|---|---|---|---|---|---|---|---|---|
| Raw Material | Atomic ratio in the charged raw material solution | Si/Al | 100 | 100 | 100 | 100 | 100 | - |
| | | Ca/Al | 2.5 | 2.5 | 2.5 | 2.5 | 2.5 | - |
| | Additive amount of seed crystal(mass%) | | 10 | 50 | 0 | 0 | 0 | - |
| Physical properties of the obtained catalyst | Atomic ratio in the synthetic zeolite catalyst | Si/Al | 100 | 110 | 120 | 100 | 110 | 70 |
| | | Ca/Al | 3.7 | 2.5 | 4.6 | 1.0 | 1.8 | - |
| | Specific surface area (m²/g) | | 320 | 320 | 310 | 320 | 340 | 420 |
| | Average particle diameter (µm) | | 1.5 | 0.8 | 4 | 8 | 3 | 0.5 |
| Evaluation of performance | Catalyst life (g-DME/g-catalyst) | | 610 | 378 | 270 | 47 | 9 | 204 |
| | C2-C4 olefin yield (mass%) | | 79 | 77 | 81 | 61 | 63 | 56 |

### [Example 2]

A proton type alkaline earth metal-containing MFI zeolite catalyst was prepared in the same way as in Example 1, with the exception of changing the additive amount of a zeolite seed crystal to 75 g (which corresponds to 50 mass% of the amount of zeolite catalyst synthesized without adding a seed crystal.). The physical-properties and performance of the catalyst were evaluated similarly to Example 1. The physical-properties and catalyst performance of the catalyst are shown in Table 1.

### [Comparative Example 1]

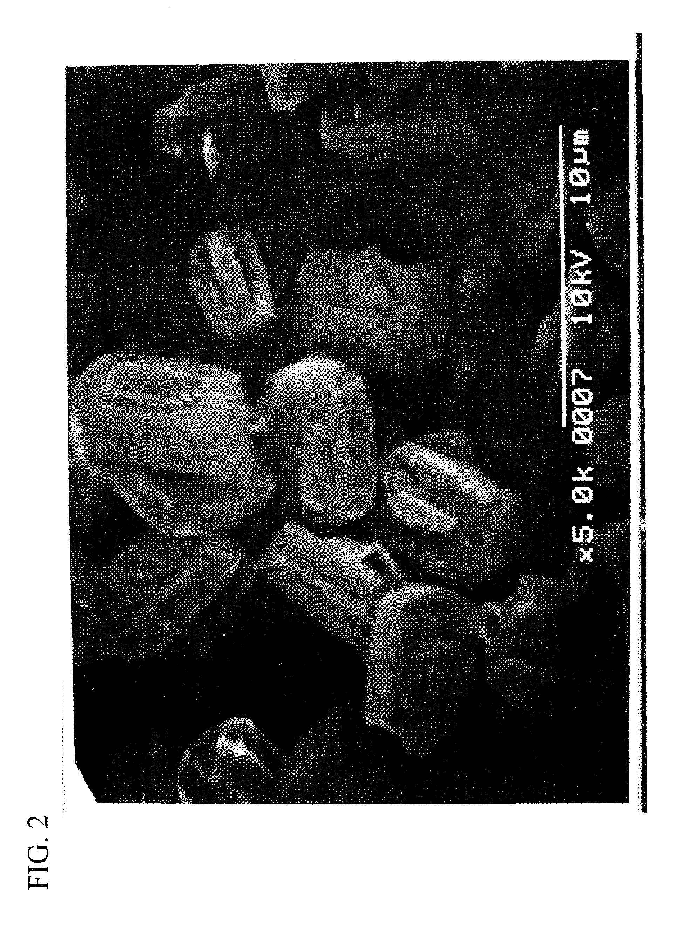

A proton type alkaline earth metal-containing MFI zeolite catalyst was prepared in the same way as in Example 1, with the exception of adding no zeolite seed crystal (similarly to the preparation example of Example 4 in Patent document 1). The physical-properties and performance of the catalyst were evaluated similarly to Example 1. The physical-properties and catalyst performance of the catalyst are shown in Table 1. In addition, a scanning electron microscopic photograph of the zeolite prepared in Comparative Example 1 is shown in FIG. 2.

### [Comparative Example 2]

To a zeolite raw material solution in which 0.13 g of Al₂(SO₄)₃ and 0.35 g of Ca(CH₃COO)₂ • H₂O were dissoleved in a 10 g of water, 12 g of 40mass% an aqueous SiO₂ solution(Snowtex40), 1.2 g of 28 mass% an aqueous NaOH solution, and 12.8 g of 17 mass% an aqueous tetrapropyl ammonium bromide solution were added while stirring to obtain an aqueous gel mixture.

Subsequently, this aqueous gel mixture was put into a small autoclave container, and agitated at 175°C under a self-pressure for 48 hours to perform hydrothermal synthesis.

By the same process as in Example 1, a proton type alkaline earth metal containing MFI zeolite catalyst was prepared. The physical-properties and performance of a catalyst were evaluated similarly to Example 1. The physical-properties and catalyst performance of the catalyst are shown in Table 1.

### [Comparative Example 3]

A proton type alkaline earth metal-containing MFI zeolite catalyst was prepared in the same way as in Comparative Example 2 with the exception of replacing the SDA with 16.2 g of 17 mass% an aqueous tetrapropyl ammonium hydroxide solution. The physical-properties and performance of the catalyst were evaluated similarly to Example 1. The physical-properties and catalyst performance of the catalyst are shown in Table 1.

### [Comparative Example 4]

A proton type MFI zeolite catalyst was prepared by calcining the ammonium type MFI zeolite (produced by Zeolyst Co., Ltd.) at 520°C which was used in Example 1 as a zeolite seed crystal. The physical-properties and performance of the catalyst were evaluated similarly to Example 1. The physical-properties and catalyst performance of the catalyst are shown in Table 1.

Comparing Examples 1 and 2 with Comparative Examples 1 to 3, in all of Examples 1 and 2 and Comparative Examples 1 to 3, the specific surface area was approximately 320 m²/g, and there were no significant differences between Examples 1 to 2 and Comparative Examples 1 to 3. On the other hand, an average particle diameter of the catalyst varied in each of the examples.

From these results, it was revealed that there was no correlation between the average particle diameter of the catalyst and the specific surface area.

Comparing Example 1 with Comparative Example 1, there were no significant differences between Example 1 and Comparative Example 1 as to the Si/Al atomic ratio and the Ca/Al atomic ratio in the synthetic zeolite catalyst. However, as is clear from FIGS. 1 and 2, the average particle diameter of Example 1 (FIG. 1) was smaller than that of Comparative Example 1 (FIG. 2), and was approximately 3/8 thereof. Moreover, the catalyst life of Example 1 was at least twice as long as that of Comparative Example 1.

Furthermore, comparing Example 2 with Comparative Example 1, the average particle diameter of Example 2 was 1/5 of that of Comparative Example 1, and the catalyst life of Example 2 was 1.4 times longer than that of Comparative Example 1. From these results, it was confirmed that the catalyst of the present invention has an average particle diameter smaller than that of well known zeolite catalysts, and a catalyst life longer than that of known known zeolite catalysts.

Comparing Examples 1 and 2 with Comparative Example 1 as to the C2-C4 olefin yield, the yield was not less than 70% and there were no significant differences therebetween.

From this result, it was confirmed that a lower unsaturated hydrocarbon can be produced at a high yield of not less than 60% through each catalyst of Examples 1 and 2.

Comparing Examples 1 and 2 with Comparative Example 4, the catalyst life of Comparative Example 4 was shorter than that of Examples 1 and 2, and the C2-C4 olefin yield of Comparative Example 4 was not more than 60%.

From these results, it was confirmed that each of the proton type alkaline earth metal-containing MFI zeolite catalysts in Examples 1 and 2 has a catalyst performance which is higher than that of the proton type MFI zeolite catalyst in Comparative Example 4, which was used as the zeolite seed crystal of Examples 1 and 2.

### INDUSTRIAL APPLICABILITY

The present invention can be applied to a zeolite catalyst used in a process for synthesizing a lower hydrocarbon by a dehydration reaction from dimethyl ether and/or methanol, a preparation process of the catalyst, and a process for producing a lower hydrocarbon using the catalyst.

## Claims

1. An alkaline earth metal-containing MFI zeolite catalyst for use in synthesizing a lower hydrocarbon from dimethyl ether and/or methanol, comprising a Si/Al atomic ratio ranging from 30 to 400, an alkaline earth metal/Al atomic ratio ranging from 0.75 to 15, and an average particle diameter ranging from 0.05 to 2 µm.

2. The alkaline earth metal-containing MFI zeolite catalyst as set forth in claim 1, wherein said alkaline earth metal-containing MFI zeolite catalyst is a proton type.

3. A process for preparing an alkaline earth metal-containing MFI zeolite catalyst for use in synthesizing a lower hydrocarbon from dimethyl ether and/or methanol, comprising synthesizing a zeolite raw material solution which contains a SiO₂ source, a metal oxide source, an alkaline source, and a structure directing agent, in the presence of an alkaline earth metal salt and a zeolite seed crystal.

4. The process for preparing an alkaline earth metal-containing MFI zeolite catalyst as set forth in claim 3, wherein the amount of said zeolite seed crystal added ranges from 1 to 60 mass% of the amount of an alkaline earth metal-containing MFI zeolite catalyst which is synthesized without adding said zeolite seed crystal.

5. The process for preparing an alkaline earth metal-containing MFI zeolite catalyst as set forth in claim 3, wherein said zeolite seed crystal has a MFI structure.

6. A process for producing a lower hydrocarbon comprising synthesizing a lower hydrocarbon from dimethyl ether and/or methanol,
wherein said alkaline earth metal-containing MFI zeolite catalyst as set forth in claim 1 is employed,
said lower hydrocarbon is an unsaturated hydrocarbon having 2 to 4 carbon atoms, and the yield of the carbon atoms contained in said lower hydrocarbon to the carbon atoms contained in said supplied dimethylether and/or said methanol is not less than 60% by weight.
